# EUROPEAN PATENT SPECIFICATION

(11) **EP 3 607 835 B1**
(45) Date of publication and mention of the grant of the patent: **12.01.2022**
(21) Application number: 17934367.8
(22) Date of filing: 15.12.2017
(51) Int. Cl.: A23L 33/105, A61Q 19/08

(54) **ANTI-AGING HEALTH FOOD AND COSMETIC AND METHOD FOR PRODUCING GRAPESEED-DERIVED ANTI-AGING COMPONENT**
ALTERUNGSHEMMENDES GESUNDES LEBENSMITTEL UND KOSMETIKUM UND VERFAHREN ZUR HERSTELLUNG EINES ALTERUNGSHEMMENDEN BESTANDTEILS AUS TRAUBENKERNEN
ALIMENT DIÉTÉTIQUE ET PRODUIT COSMÉTIQUE ANTIVIEILLISSEMENT, ET PROCÉDÉ DE PRODUCTION D'UN CONSTITUANT ANTIVIEILLISSEMENT DÉRIVÉ DE PÉPINS DE RAISIN

(43) Date of publication of application: 12.02.2020
(73) Proprietor: NAGAO, Tsukasa, Nagahama-shi Siga 526-0015 (JP)
(72) Inventor: NAGAO, Tsukasa, Nagahama-shi Siga 526-0015 (JP)
(74) Representative: Isern Patentes y Marcas S.L.
(86) International application number: PCT/JP2017/045207
(87) International publication number: WO 2019/116574

(56) References cited:
- WO-A1-2006/113700
- WO-A1-2014/140312
- WO-A2-2007/049796
- CN-A- 103 509 642
- JP-A- 2007 145 809
- JP-A- 2011 529 909
- JP-A- 2014 073 988
- JP-A- 2014 073 988
- ARIGA, Toshiaki et al.: "Studies on functional properties of proanthocyanidins and their practical application", Nippon Nogeikagaku Kaishi, vol. 74, no. 1, 2000, pages 1-8, XP055617529,

## Description

### [BACKGROUND OF THE INVENTION]

### [FIELD OF THE INVENTION]

The present invention relates to anti-aging health food and cosmetics, and a method of manufacturing a grape seed-derived anti-aging ingredient, in particular, to anti-aging health food and cosmetics having better anti-aging effect than resveratrol and a method of manufacturing a grape seed-derived anti-aging ingredient having the better anti-aging effect.

### [BACKGROUND]

Previous study about aging prevention and life control has revealed that a compound, for example, resveratrol (trans-3, 4', 5-trihydroxy-stillbene) is expected to have anti-aging effect.

For example, Konrad T. Howitz et al., Nature425, and 191-196 (2003) reports that in a budding yeast (Saccharomyces cerevisiae), resveratrol stimulated Sir2 of an enzyme of Caenorhabditis elegans adjusting a life span in order to improve DNA stability of the budding yeast, thereby extending the life span of the budding yeast by 70%.

However, as described in Richard A. Miller et al., Journal of Gerontology: BIOLOGICAL SCIENCES 191-201 (2010), it has been reported that resveratrol does not extend the life span of a male and female mouse nor have any anti-aging effect.

JP2014073988A, descries a functional composition containing grape seed extract powder, said composition having aging odor inhibiting action, anticancer action and visual function improvement action. Said composition can also be used to prepare health food and a cosmetic containing the composition. JP2014073988A, also described a production method to obtained said compositions.

Moreover, JP2014073988A, described that grape seed extract powders extracted from grape seeds treated with water have an effect of increasing Mitochondrial biogenesis thereby suppressing age-related odor. However, JP2014073988A is silent regarding any effect the grape seed extract powders may have in increasing the amount of apoptosis cells and/or decreasing the amount of necrosis cells. Likewise, JP2014073988A did not mention nor suggested any process for forcing sprouting.

Furthermore, other publications, such as WO 2014/140312 A1, described oral compositions for pharmaceutical or nutritional or cosmetic use, having antioxidant activity against free radicals as well as the process to obtain said composition from Vitis vinifera seed, or seed and leaf extract containing a combination of the flavonoids catechin using in different molar ratios together with b) an olive, Olea europaea L, leaf extract having a hydroxytyrosol.

Accordingly, while resveratrol is expected to have anti-aging effect, it has not been confirmed whether resveratrol actually has the anti-aging effect.

### [SUMMARY OF THE INVENTION]

As described above, it has not been confirmed whether resveratrol expected to have anti-aging effect actually has such anti-aging effect.

Accordingly, there is a need for a substance having more reliable and better anti-aging effect than resveratrol.

After earnest consideration, the inventors found that an extract consisting of grape seed-derived polyphenol had better anti-aging effect than resveratrol.

The problem to be solved by the present invention is to provide a grape seed-derived anti-aging ingredient having better anti-aging effect than resveratrol expected to have such anti-aging effect, and a method of manufacturing such a grape seed-derived anti-aging ingredient, and to provide anti-aging health food and an anti-aging cosmetics containing the anti-aging ingredient.

### [MEANS FOR SOLVING THE PROBLEMS]

The present invention according to a first aspect relates to an anti-aging health food and cosmetics, comprising a grape seed-derived anti-aging ingredient consisting of 60 wt. % or more of crudely-purified grape seed-derived polyphenol.

The present invention according to a second aspect relates to a method of manufacturing a grape seed-derived anti-aging ingredient, comprising:
(Step 1) pretreating one or more grape seeds selected from a group consisting of Vitis vinifera L., Vitis labrusca L., Vitis coignetiae L., Vitis amurensis L., and Vitis shiragai L. to force sprouting of these grape seeds and drying sprout-forcing grape seeds at 35 °C - 60 °C;
(Step 2) powdering the grape seeds dried in the Step 1;
(Step 3) obtaining an extracted fraction containing grape seed-derived polyphenol by immersing the powdered grape seeds obtained in the Step 2 in either of water, ethanol, or a mixed solvent of water and ethanol; and
(Step 4) drying and powdering the extracted fraction containing the grape seed-derived polyphenol obtained in the Step 3.

The present invention according to a third aspect relates to the anti-aging health food and cosmetics of the first aspect or the method of the second aspect, wherein the grape seed is a seed of one or more wine grape cultivars selected from a group consisting of: Agiorgitiko, Viognier, CabernetSauvignon, Cabernet Franc, Gamay, Carignan, Carmenere, Xinomavro, Grenache, Gewurztraminer, Kerner, Colombard, Koshu, Sultana, Sangiovese, Chardonnay, Chenin Blanc, Syrah, Zinfandel, Semillon, Sauvignon Blanc, Tannat, Zweigelt, Tempranillo, Trebbiano, Nebbiolo, Nero D'Avola, Barbera, Pinotage, Pinot Noir, Pinot Gris, Pinot Blanc, Petit Verdot, Black Queen, Muscat Bailey A, Malbec, Muller-Thurgau, Mourvedre, Meunier, Melon de Bourgogne, Merlot, Moscato, Yama-Sauvignon, Riesling, and Ruby Cabernet.

### [Effect of the present invention]

According to the present invention of the first aspect, the anti-aging health food and cosmetics comprises a grape seed-derived anti-aging ingredient consisting of 60 wt. % or more of crudely-purified grape seed-derived polyphenol. Thus, an anti-aging health food and cosmetics having good anti-aging effect can be provided.

According to the present invention of the second aspect, the grape seed-derived anti-aging ingredient is manufactured by (Step 1) pretreating one or more grape seeds selected from a group consisting of Vitis vinifera L., Vitis labrusca L., Vitis coignetiae L., Vitis amurensis L., and Vitis shiragai L. to force sprouting of these grape seeds and drying sprout-forcing grape seeds at 35 °C - 60 °C;
(Step 2) powdering the grape seeds dried in the Step 1;
(Step 3) obtaining an extracted fraction containing grape seed-derived polyphenol by immersing the powdered grape seeds obtained in the Step 2 in either of water, ethanol, or a mixed solvent of water and ethanol; and
(Step 4) drying and powdering the extracted fraction containing the grape seed-derived polyphenol obtained in the Step 3. Thus, a grape seed-derived anti-aging ingredient having good anti-aging effect can be manufactured.

According to the present invention of the third aspect, the grape seed used for the anti-aging health food and cosmetics of the first aspect or for the method of the second aspect is a seed of one or more wine grape cultivars selected from a group consisting of Agiorgitiko, Viognier, CabernetSauvignon, Cabernet Franc, Gamay, Carignan, Carmenere, Xinomavro, Grenache, Gewurztraminer, Kerner, Colombard, Koshu, Sultana, Sangiovese, Chardonnay, Chenin Blanc, Syrah, Zinfandel, Semillon, Sauvignon Blanc, Tannat, Zweigelt, Tempranillo, Trebbiano, Nebbiolo, Nero D'Avola, Barbera, Pinotage, Pinot Noir, Pinot Gris, Pinot Blanc, Petit Verdot, Black Queen, Muscat Bailey A, Malbec, Muller-Thurgau, Mourvedre, Meunier, Melon de Bourgogne, Merlot, Moscato, Yama-Sauvignon, Riesling, and Ruby Cabernet. Thus, an anti-aging health food and cosmetics having better anti-aging effect can be manufactured, or a grape seed-derived anti-aging ingredient having better anti-aging effect can be manufactured.

### [BRIEF DESCRIPTION OF DRAWINGS]

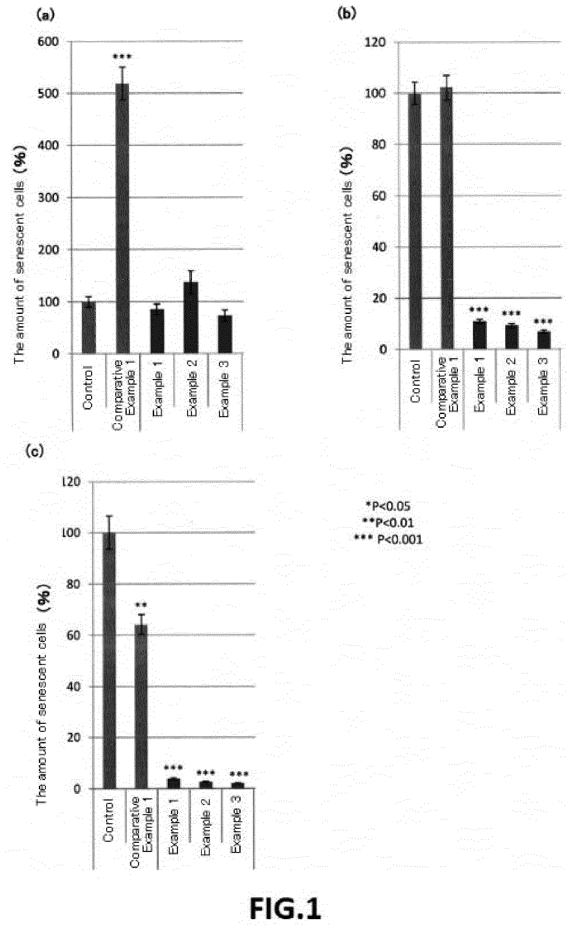

[Fig. 1] Fig. 1 is a graph showing the amount of senescent cells calculated in Examples 1-3, Comparative Example 1, and a Control. (a) shows the amount of the senescent cells in cells cultured for 7 days, (b) shows the amount of the senescent cells in cells cultured for 14 days, and (c) shows the amount of the senescent cells in cells cultured for 21 days. Further, assuming that the amount of the senescent cell calculated in a Control is 100 %, the amounts of the senescent cells in Examples 1-3 and Comparative Example 1 are shown in a ratio to the amount of senescent cell in the Control.

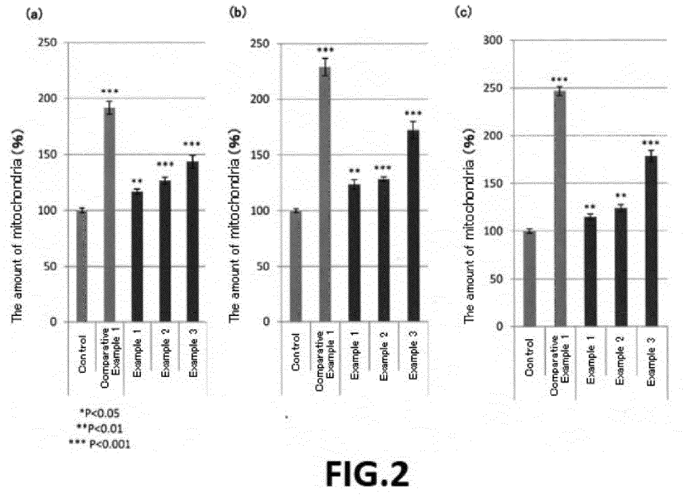

[Fig. 2] Fig.2 is a graph showing the amount of mitochondria measured in Examples 1-3, Comparative Example 1, and a Control. (a) shows the amount of the mitochondria in cells cultured for 7 days, (b) shows the amount of the mitochondria in cells cultured for 14 days, and (c) shows the amount of the mitochondria in cells cultured for 21 days. Further, assuming that the amount of the mitochondria calculated in a Control is 100 %, the amounts of the mitochondria in Examples 1-3 and Comparative Example 1 are shown in a ratio to the amount of mitochondria in the Control.

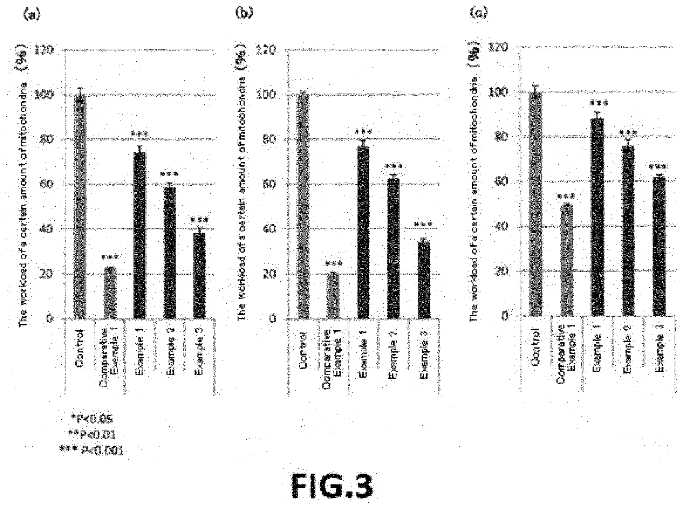

[Fig. 3] Fig.3 is a graph showing the workload of a certain amount of mitochondria measured in Examples 1-3, Comparative Example 1, and a Control. (a) shows the workload of a certain amount of the mitochondria in cells cultured for 7 days, (b) shows the workload of a certain amount of the mitochondria in cells cultured for 14 days, and (c) shows the workload of a certain amount of the mitochondria in cells cultured for 21 days. Further, assuming that the workload of a certain amount of the mitochondria calculated in a Control is 100 %, the workload of a certain amount of the mitochondria in Examples 1-3 and Comparative Example 1 is shown in a ratio to the workload of a certain amount of mitochondria in the Control.

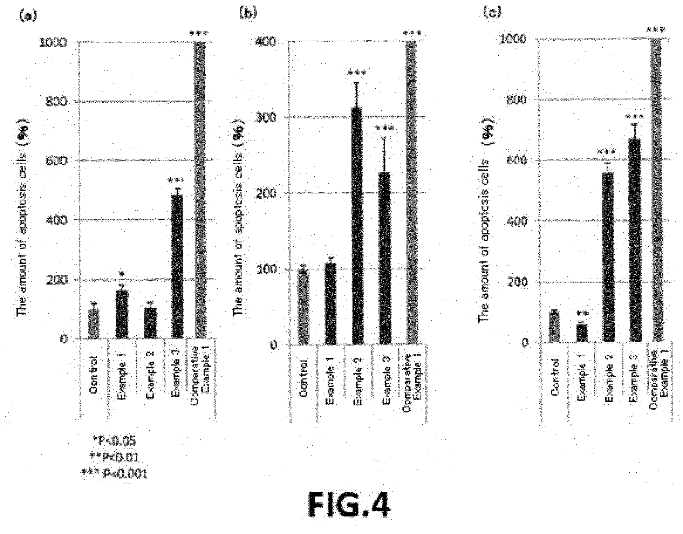

[Fig. 4] Fig.4 is a graph showing the amount of apoptosis cells measured in Examples 1-3, Comparative Example 1, and a Control. (a) shows the amount of the apoptosis cells in cells cultured for 7 days, (b) shows the amount of the apoptosis cells in cells cultured for 14 days, and (c) shows the amount of the apoptosis cells in cells cultured for 21 days. Further, assuming that the amount of the apoptosis cells calculated in a Control is 100 %, the amounts of the apoptosis cells in Examples 1-3 and Comparative Example 1 are shown in a ratio to the amount of the apoptosis cells in the Control.

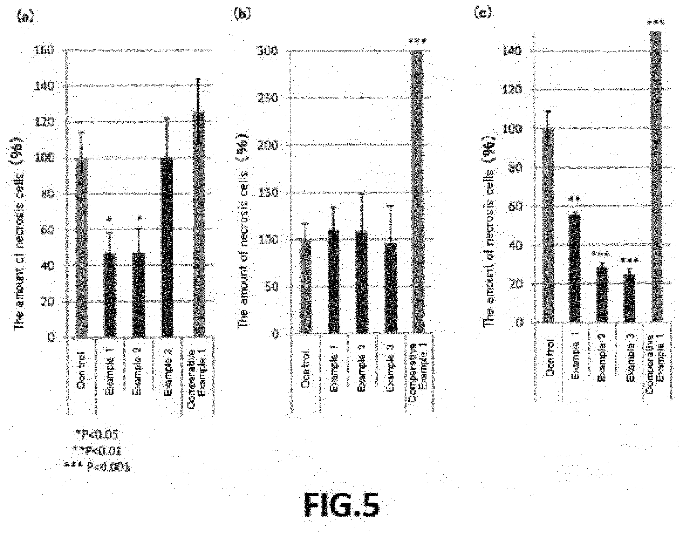

[Fig. 5] Fig.5 is a graph showing the amount of necrosis cells measured in Examples 1-3, Comparative Example 1, and a Control. (a) shows the amount of the necrosis cells in cells cultured for 7 days, (b) shows the amount of the necrosis cells in cells cultured for 14 days, and (c) shows the amount of the necrosis cells in cells cultured for 21 days. Further, assuming that the amount of the necrosis cells calculated in a Control is 100 %, the amounts of the necrosis cells in Examples 1-3 and Comparative Example 1 are shown in a ratio to the amount of the necrosis cells in the Control.

### [DETAILED DESCRIPTION OF THE INVENTION]

Hereinafter, an anti-aging health food and cosmetics, and a method of manufacturing a grape seed-derived anti-aging ingredient according to the present invention will be set forth.

Further, in this specification, "aging" refers to a state where doubling ability of a cell decreases or a state where a cell irreversibly stops growing in G1 phase. Suppression of a gene facilitating cell cycle progression, and increased expression of p16^{INK4a} and p53 genes inhibiting cell cycle and their targeting transcription factor, p21^{CIP1}, are involved in these states. In addition, a senescent cell is resistant to mitogen-induced cell proliferation, and thus grows huge and flattens. The senescent cell exhibits a common biochemical marker, such as a senescence-associated β-galactosidase (SA β Gal) activity. While this aging phenomenon has been characterized by a cultured cell, it has been proved to occur even in a living body.

The anti-aging health food and cosmetics according to the present invention contain a grape seed-derived anti-aging ingredient consisting of 60 wt. % or more of crudely-purified grape seed-derived polyphenol.

The grape seed-derived anti-aging ingredient consisting of grape seed-derived polyphenol contained in the anti-aging health food and cosmetics according to the present invention is obtained from following manufacturing steps.

### <Step 1>

Step 1 is pretreating a grape seed to force sprouting and drying the sprout-forcing grape seed.

The type of grape for the grape seed used in the present invention is, but not limited to, for example, one or more grapes selected from a group consisting of Vitis vinifera L., Vitis labrusca L., Vitis coignetiae L., Vitis amurensis L., and Vitis shiragai L.

Among others, preferable grape is a wine grape cultiber, such as Agiorgitiko, Viognier, Cabernet Sauvignon, Cabernet Franc, Gamay, Carignan, Carmenere, Xinomavro, Grenache, Gewurztraminer, Kerner, Colombard, Koshu, Sultana, Sangiovese, Chardonnay, Chenin Blanc, Syrah, Zinfandel, Semillon, Sauvignon Blanc, Tannat, Zweigelt, Tempranillo, Trebbiano, Nebbiolo, Nero D'Avola, Barbera, Pinotage, Pinot Noir, Pinot Gris, Pinot Blanc, Petit Verdot, Black Queen, Muscat Bailey A, Malbec, Muller-Thurgau, Mourvedre, Meunier, Melon de Bourgogne, Merlot, Moscato, Yama-Sauvignon, Riesling, and Ruby Cabernet. In particular, Cabernet Sauvignon, Merlot, Syrah, and Pinot Noir are more preferable.

In this specification, "sprout-forcing" means forcing a grape seed into the state in which the seed begins to sprout. More specifically, it means the state in which the embryo portion of the grape seed slightly bulged and swelled.

The degree of the bulge is, but not limited to, for example, the state in which the surface of the embryo portion bulged 1-2 mm higher than the grape seed before sprout-forcing.

In addition, in the present invention, the grape seed in a sprout state in which the grape seed forms a bud is not preferable because it has a low tendency for an anti-aging effect compared with the grape seed in a sprout-forcing state.

In the present invention, a method of pretreating a grape seed to force sprouting of the grape seed includes, but not limited to, any methods conventionally used for forcing sprouting of plant seeds, for example, physical methods such as low-temperature processing, warm bath processing and mechanical destruction, and chemical methods such as gas (acetylene, ether, hydrogen gas, etc.) processing, auxin processing and gibberellin processing.

As described above, in the present invention, the method of pretreating a grape seed to force sprouting of the grape seed is not particularly limited. For example, more specifically, it is possible to force sprouting of the grape seed by the following pretreatment method.

Firstly, a grape seed is immersed in water at 30-60 °C.

The immersion time is preferably, but not limited to, 20 to 80 hours.

Secondly, the grape seed immersed in water at 30-60 °C is removed from the water, and naturally dried in the air. The natural drying temperature is preferably, but not limited to, 10 to 50 °C. The natural drying time is preferably, but not limited to, 1 to 10 hours.

Then, the grape seed naturally dried in the air is immersed in water at 15-45 °C.

The immersion time is preferably, but not limited to, 10 to 200 minutes.

Secondly, the grape seed immersed in water at 15-45 °C is removed from the water, and naturally dried in the air.

The natural drying temperature is preferably, but not limited to, 10 to 50 °C.

The natural drying time is preferably, but not limited to, 3 to 12 hours.

The step of immersing the grape seed in water at 15-45 °C and the step of naturally drying it in the air (i.e., intermittently immersing the grape seed into water) are repeated until the embryo portion of the grape seed slightly bulged and swelled after the natural drying.

When the embryo portion of the grape seed slightly bulged and swelled after natural the drying, the grape seed is removed and further dried for 2 to 5 days at the temperature sufficient to sterilize various germs (80 °C or less).

This drying time may be appropriately changed depending on season, ambient temperature, or humidity.

This stimulation of sprout-forcing processing exfoliates phenol molecule from a thin phenol layer in the grape seed. Then, the molecule multiply bonded to produce polyphenol.

Although a resveratrol, a type of polyphenol, has a molecular weight of about 250, this newly produced ingredient is characterized by a large polymer structure containing the ingredient of the molecular weight of 4000.

### <Step 2>

Step 2 is powdering the grape seed in a sprout-forcing state dried in the step 1.

The method of powdering can utilize, but not limited to, standard methods such as powdering with a mill.

### <Step 3>

Step 3 is extracting the powdered grape seed with a solvent.

By this step 3, it is possible to obtain a grape seed-derived anti-aging ingredient consisting of grape seed-derived polyphenol.

Water, ethanol, or a mixed solvent of water and ethanol can be used as a solvent.

The extraction may be performed by adding a solvent of 50-1000 parts by weight to the powdered grape seed of 100 parts by weight.

As described above, the grape seed-derived anti-aging ingredient consisting of grape seed-derived polyphenol contains a polymer ingredient with a molecular weight of around 4000 (3500-4500).

### <Step 4>

Then, the grape seed-derived anti-aging ingredient consisting of grape seed-derived polyphenol obtained in the step 3 is dried and provided.

A drying method preferably uses, but not limited to, decompression drying.

The method of powdering can utilize, but not limited to, standard methods such as powdering with a mill.

The above steps can provide a grape seed-derived anti-aging ingredient consisting of polyphenol derived from a powdered grape seed.

The grape seed-derived anti-aging ingredient obtained by the above steps contains 50-80 wt. % of crudely-purified polyphenol.

From the viewpoint of providing a remarkable anti-aging effect, the grape seed-derived anti-aging ingredient desirably contains 60 wt. % or more of crudely-purified polyphenol.

The polyphenol contained in the grape seed-derived anti-aging ingredient is polyphenol contained in the seed of each grape cultivar, for example, resveratrol, tannin, or the like.
50-80 wt. % of the polyphenol contained in the grape seed-derived anti-aging ingredient is proanthocyanidin polymer.

The inventors confirmed that the grape seed-derived anti-aging ingredient containing the above ingredient provides an anti-aging effect.

The term, "crudely-purified", herein means a state in which an extract is dried and powdered only, foreign substances are contained, and the processing such as concentration is not performed. The grape seed-derived anti-aging ingredient obtained in the steps 1-4 is crudely-purified.

Containing the grape seed-derived anti-aging ingredient in health food can provide the anti-aging health food of the present invention.

With the grape seed-derived anti-aging ingredient contained in health food, it is possible to take the grape seed-derived anti-aging ingredient readily and routinely.

The anti-aging health food of the present invention can be manufactured in an usual manner by appropriately adding additive agents used as a common food raw material, for example, glucose, fructose, sucrose, maltose, sorbitol, stevioside, rubusoside, corn syrup, lactose, citrate acid, tartaric acid, malic acid, succinic acid, lactic acid, L-ascorbic acid, dl-alpha-tocopherol, sodium erythorbate, glycerin, propylene glycol, glycerol fatty acid ester, polyglycerol fatty acid ester, sucrose fatty acid ester, sorbitan fatty acid ester, propylene glycol fatty acid ester gum arabic, carragheenan, casein, gelatin, pectin, agar, vitamin B, nicotinic acid amide, calcium pantothenate, amino acid, calcium salt, pigment, flavoring agent, and preservative.

Containing the grape seed-derived anti-aging ingredient in cosmetics can provide the anti-aging cosmetics of the present invention.

With the grape seed-derived anti-aging ingredient contained in cosmetics, it is possible to apply the grape seed-derived anti-aging ingredient routinely.

The form of a grape seed-derived anti-aging ingredient contained in the anti-aging cosmetics according to the present invention is preferably, but not limited to, a microcapsule having a multi-layer, from the viewpoint of ease of permeating the skin.

The anti-aging cosmetics of the present invention can be manufactured in a usual manner by appropriately adding additive agents commonly used, such as whitening agent, active oxygen remover, antioxidant, ultraviolet inhibitor, etc.

The intake of the grape seed-derived anti-aging ingredient in the anti-aging health food and cosmetics of the present invention is preferably, but not limited to, at least 50 mg or more intake per day, from the viewpoint of providing a remarkable anti-aging effect.

If the intake of the grape seed-derived anti-aging ingredient in the anti-aging health food and cosmetics of the present invention is less than 50 mg per day, the anti-aging effect cannot be exerted sufficiently, and thus, the intake is not desirable.

Moreover, even if the intake of the grape seed-derived anti-aging ingredient in the anti-aging health food and cosmetics of the present invention is more than 7500 mg per day (per body weight of 60 kg), the rate of increase of the effect is small.

Therefore, the intake of the grape seed-derived anti-aging ingredient in the anti-aging health food and cosmetics of the present invention is preferably 50-7500 mg per day.

Depending on application, an excipient, a pH adjuster, a preservative, a chelating agent, a stabilizer or the like may be suitably added to the anti-aging health food and cosmetics of the present invention.

Moreover, the intake of the grape seed-derived anti-aging ingredient contained in the anti-aging health food and cosmetics of the present invention is preferably, but not limited to, for example, 0.8-125mg/kg/day, from the viewpoint of providing a remarkable anti-aging effect.

If the intake of the grape seed-derived anti-aging ingredient in the anti-aging health food and cosmetics of the present invention is less than 0.8 mg/kg/day, the anti-aging effect cannot be exerted sufficiently, and thus, the intake is not desirable.

Also, even if the intake of the grape seed-derived anti-aging ingredient in the anti-aging health food and cosmetics of the present invention is more than 125 mg/kg/day, further effect cannot be exerted.

Therefore, the intake of the grape seed-derived anti-aging ingredient in the anti-aging health food and cosmetics of the present invention is preferably 0.8-125 mg/kg/day.

### [EXAMPLE]

The anti-aging health food and cosmetics, and the grape seed-derived anti-aging ingredient according to the present invention are described in detail based on the following Examples, but a method of manufacturing the anti-aging health food and cosmetics, and the grape seed-derived anti-aging ingredient according to the present invention is not limited to the Examples.

### <Example 1: Manufacture of a grape seed-derived anti-aging ingredient>

A seed of Cabernet Sauvignon was immersed in water at about 40 °C for 45 to 60 hours, then this grape seed was removed from the water and naturally dried in the air at room temperature (about 25 °C) for 3 to 5 hours (stored in a room).

The naturally dried grape seed was immersed in water at 25-30 °C for 60 to 80 minutes, then this grape seed was removed from the water and naturally dried in the air at room temperature (about 25 °C) for 3 hours (stored in a room).

The immersion in water at 30 °C and the natural drying for 3 hours were repeated 3 times, then the dried grape seed was observed. It confirmed that about 5-15% of the embryo portion of the grape seed bulged about 1 mm. The above-mentioned procedures are referred to as sprout-forcing processing.

The sprout-forcing proceeding was ended when the bulge of the embryo portion of grape seed was confirmed, and the grape seed was further dried at 45 °C for 3 days with a far infrared radiation drier.

After drying the grape seed for 3 days, the seed was powdered with a mill to produce grape seed powder.

Subsequently, the grape seed powder of 50 parts by weight was added to water of 100 parts by weight, and the fraction dissolved in water was extracted to provide a grape seed-derived anti-aging ingredient.

After the decompression drying of the obtained grape seed-derived anti-aging ingredient, it was powdered with a mill to provide a powdered grape seed-derived anti-aging ingredient.

The total amount of polyphenol contained in the powdered grape seed-derived anti-aging ingredient was quantified.

The quantification was performed using the official method of AOAC International (AOAC official method 952.03, 15th Ed) (also referred to as Folin-Denis method).

The Folin-Denis method quantifies the total amount of polyphenol by measuring blue color (wavelength of 700-770 nm) with a spectrophotometer, wherein the blue color is obtained by reducing phosphotungstic acid and molybdic acid with phenolic hydroxyl in alkaline condition.

As a result of the quantification, it was found that the grape seed-derived anti-aging ingredient contains 69 wt. % of polyphenol.

Also, it was confirmed that 71 wt. % of the polyphenol contained in the grape seed-derived anti-aging ingredient (i.e., 49 wt. % of the grape seed-derived anti-aging ingredient) was a proanthocyanidin polymer.

Hereinafter, the grape seed-derived anti-aging ingredient prepared according to this Example is referred to as an Example, and the resveratrol (manufactured by SIGMA, product No.: R5010) used as a comparison target is referred to as a Comparative Example.

In the following Examples 3-5, the anti-aging effect of the grape seed-derived anti-aging ingredient according to the present invention was examined.

### <Example 2: Preparation of normal human fibroblasts, Example, and Comparative Example>

### • Preparation of normal human fibroblasts

Normal human fibroblasts for each test were prepared at first.

Subsequent to initiation, Normal human fibroblasts (KURABO INDUSTRIES LTD., product No.: KF-4109) were cultured in a basal medium (DMEM (Nacalai Tesque, Inc., product No.: 08456-65)), 10% FBS (BioWest, product No.:S1820, Lot No.516536), and 1% antibiotic (penicillin-streptomycin mixed solution (Nacalai Tesque, Inc., product No.:26253-84)) in a CO₂ incubator (5% CO₂, 37°C) until a required number of cells were obtained.

After the culture, the normal human fibroblasts were released by trypsin/EDTA (2.5g/1-trypsin/1mmol/1-EDTA solution (Nacalai Tesque, Inc., product No.:32777-44), and used for each test after calculating the number of cells.

### • Preparation of Example and Comparative Example

Example and Comparative Example were then prepared.

Example and Comparative Example were measured respectively and resolved in a basal medium to become a concentration of 1% (w/v).

After the resolution, Example and Comparative Example resolved in a basal medium were centrifuged respectively (120,000rpm (2,000s⁻¹), 10 minutes) to remove insoluble materials.

After the centrifugal separation, supernatant was collected, filtered and sterilized through a sterilizing filter to be used for each test.

### <Example 3: Test for confirming anti-aging effect>

Anti-aging effect of Example and Comparative Example was tested by measuring an activity of β-galactosidase (senescence-associated β-galactosidase; SA-β-gal) generally used as a cell aging marker.

Overexpression of SA-β-gal was confirmed in senescent cells. It has been confirmed that an aging process of the cells measured by the aging marker occurs not only in cultured cells but also in an organism.

The amount of the senescent cells recognized by the test for confirming anti-aging effect most specifically indicates anti-aging effect, as a comprehensive parameter.

### (Test procedure)

Normal human fibroblasts prepared with a basal medium was seeded on a 96-well plate so as to be 1×10⁴ cells/0.1ml/well (black plate for fluorescence observation (Sumitomo Bakelite Co., Ltd. product No.: MS-8096F)) and cultured for 24 hours.

After the culture, culture supernatants were respectively replaced with the below mediums with different conditions, and normal human fibroblasts were cultured again.

The conditions for each medium are as follows. Each cell cultured with mediums with each condition are referred to as Examples 1-3, Comparative Example 1, and Control respectively.
Example 1: a basal medium comprising 0.002 wt% of grape seed-derived anti-aging ingredients of the present invention.
Example 2: a basal medium comprising 0.01 wt% of grape seed-derived anti-aging ingredients of the present invention.
Example 3: a basal medium comprising 0.05 wt% of grape seed-derived anti-aging ingredients of the present invention.
Comparative Example 1: a basal medium containing 0.0023 wt% of resveratrol. Control: a basal medium (comprising no grape seed-derived anti-aging ingredients of the present invention nor resveratrol).

Hereinafter, the above-mentioned Examples 1-3, Comparative Example 1, and Control were also used in Examples 4-5.

The concentration of resveratrol in Comparative Example 1 was set to be 0.0023 wt%, because necrosis cells increase too much so that a correct measurement would be difficult in a necrosis measuring test mentioned below if the concentration of resveratrol is set to be more than 0.0023 wt%.

Normal human fibroblasts were cultured respectively with the conditions for up to 21 days.

The mediums were exchanged every 2 to 3 days.

After the culture, culture supernatants were removed and the cells were washed twice with PBS (Nissui Pharmaceutical Co., Ltd., product No.: 05913).

To the washed normal human fibroblasts were added a basal medium containing a 1000-fold diluted Bafilomycin, and the normal human fibroblasts were cultured at 37°C for 60 minutes.

To the culture solution was then added a basal medium comprising a 1000-fold diluted SPiDER-β Gal working solution and a 100-fold diluted Hoechist 33342 solution (DOJINDO LABORATORIES, product No.: 346-07951) as a nuclei staining reagent, and normal human fibroblasts were further cultured at 37°C for 30 minutes.

After the culture, normal human fibroblasts were washed in a basal medium.

After wash, the basal medium was added to normal human fibroblasts, and nuclei staining (living cells) and stained images of the senescent cells were taken by a fluorescence microscope (Keyence Corporation, product No.: BZ-X7000).

The amount of the senescent cells were calculated based on the respective fluorescent images.

The Bafilomycin A1 and SPiDER-β Gal working solution were from Cellular Senescence Detection Kit-SPiDER-βGal (DOJINDO LABORATORIES, product No.: SG03).

### (Results)

Fig.1 shows a graph indicating the amount of the senescent cells calculated for Examples 1-3, Comparative Example 1, and Control.

In Fig.1, (a) shows the amount of the senescent cells among cells cultured for 7 days, (b) shows the amount of the senescent cells among cells cultured for 14 days, and (c) shows the amount of the senescent cells among cells cultured for 21 days.

In Fig.1, assuming that the amount of the senescent cells in a Control is 1 00%, the amounts of the senescent cells in Examples 1-3 and Comparative Exam ple 1are shown in a ratio to the amount of the senescent cell in the Control.

The amount of the senescent cells in Comparative Example 1 cultured for 7 days was 519% relative to Control (see Fig. 1(a)).

The amount of the senescent cells in Example 1 cultured for 7 days was 85%, the amount of the senescent cells in Example 2 was 137%, and the amount of the senescent cells in Example 3 was 73%, relative to Control (see Fig. 1(a)).

The amount of the senescent cells in Comparative Example 1 cultured for 14 days was 102% relative to Control (see Fig. 1(b)).

The amount of the senescent cells in Example 1 cultured for 14 days was 11%, the amount of the senescent cells in Example 2 was 9%, and the amount of the senescent cells in Example 3 was 7%, relative to Control (see Fig. 1(b)).

The amount of the senescent cells in Comparative Example 1 cultured for 21 days was 64% relative to Control (see Fig. 1(c)).

The amount of the senescent cells in Example 1 cultured for 21 days was 4%, the amount of the senescent cells in Example 2 was 3%, and the amount of the senescent cells in Example 3 was 2%, relative to Control (see Fig. 1(c)).

According to these results, it turned out that resveratrol had more senescent cells among cells cultured for 7 days than Control and promoted aging of cells. Therefore, it is considered that resveratrol has an effect of promoting aging in the short term.

Further, it turned out that the amount of the senescent cells in culture Day 21 cells were suppressed around 64% relative to Control, thus suppressing aging of cells.

While the grape seed-derived anti-aging ingredients of this invention represented almost same amount of the senescent cells as Control in cells cultured for 7 days on average, it suppressed the amount of the senescent cells in culture Day 21 cells about 2% to 4% relative to Control, thus suppressing aging of cells much more than resveratrol did.

Therefore, the grape seed-derived anti-aging ingredients has better anti-aging effect than resveratrol, and anti-aging health food and cosmetics comprising this grape seed-derived anti-aging ingredients also have a remarkable anti-aging effect.

### <Example 4: Test for measuring the amount of mitochondria and a workload of a certain amount of mitochondria>

Following the test for confirming anti-aging effect, an effect of Example and Comparative Example to the amount of mitochondria and a workload of a certain amount of mitochondria in cells was confirmed.

Conventionally, it has been considered that resveratrol has an effect on the amount of mitochondria or a workload of a certain amount of mitochondria to provide its anti-aging effect. Hence, the effect of the grape seed-derived anti-aging ingredients on the amount of mitochondria or a workload of a certain amount of mitochondria was confirmed.

The amount of mitochondria was measured using Mito Tracker Mitochondrion-Selective Probes (Invitrogen, product No.: M7512) which can selectively label mitochondria in a cell.

The workload of a certain amount of mitochondria was measured by MTT assay. Since the only target of MTT assay is dehydrogenase activity of mitochondria, the result of MTT assay reflects the workload of a certain amount of mitochondria.

### (Test Procedure)

After removing culture supernatants of normal human fibroblasts cultured in Example 2, normal human fibroblasts were washed twice with PBS (Nissui Pharmaceutical Co., Ltd., product No.: 05913).

To the washed cells were added a basal medium comprising a 1000-fold diluted Hoechist 33342 solution (DOJINDO LABORATORIES, product No.: 346-07951) and a 2000-fold diluted Mito Tracker Mitochondrion-Selective Probes (Invitrogen, product No.: M7512), and normal human fibroblasts were cultured at 37°C for 30 minutes.

After the culture, culture supernatants were removed and normal human fibroblasts were washed twice with PBS (Nissui Pharmaceutical Co., Ltd., product No.: 05913).

After wash, PBS (Nissui Pharmaceutical Co., Ltd., product No.: 05913) was newly added to the normal human fibroblasts, and nuclei staining (living cells) and stained images of mitochondria were taken by a fluorescence microscope (Keyence Corporation, product No.: BZ-X7000).

Subsequently, each fluorescence intensity was measured using a fluorescent plate reader (Thermo Fisher Scientific, product No.: VARIOSKAN FLASH) (Hoechis: excitation wavelength 356nm, fluorescence wavelength 465nm, Mito Tracker: excitation wavelength 579nm, fluorescence wavelength 599nm).

After measuring the fluorescence intensity, supernatants were removed, and normal human fibroblasts were washed once with PBS (Nissui Pharmaceutical Co., Ltd., product No.: 05913).

After the normal human fibroblasts were washed, they were replaced with PBS (100µl/well) comprising 0.5mg/ml of MTT (Thiazolyl Blue Tetrazolium Bromide (SIGMA, product No.: M5655-1G)) and reacted at 37°C for 5 hours.

Subsequently, 100µl of 0.01M HCl (SIGMA, product No.: 13-1690) and 10% SDS (Wako Pure Chemical Corporation, product No.: 191-07145) were added to each well and reacted at room temperature for 24 hours.

After the reaction, formazan of MTT was resolved.

After resolving formazan, absorbance was measured using a plate reader (Thermo Fisher Scientific, product No.: VARIOSKAN FLASH) (measurement wavelength 550nm, reference wavelength 660nm).

### (Results)

Fig.2 shows a graph indicating the amount of mitochondria measured in Examples 1-3, Comparative Example 1, and Control.

In Fig.2, (a) shows the amount of mitochondria in cells cultured for 7 days, (b) shows the amount of mitochondria in cells cultured for 14 days, and (c) shows the amount of mitochondria in cells cultured for 21 days.

In Fig.2, assuming that the amount of the mitochondria in a Control is 100 %, the amounts of the mitochondria in Examples 1-3 and Comparative Example 1 are shown in a ratio to the amount of mitochondria in the Control.

In the Comparative Example 1 cultured for 7 days, the amount of mitochondria was 191% relative to Control (see Fig. 2(a)).

The amount of mitochondria in Example 1 cultured for 7 days was 116% relative to Control, the amount of mitochondria in Example 2 was 126%, and the amount of mitochondria in Example 3 was 143% (see Fig. 2(a)).

The amount of mitochondria in Comparative Example 1 cultured for 14 days was 228% relative to Control (see Fig. 2(b)).

The amount of mitochondria in Example 1 cultured for 14 days was 123%, the amount of mitochondria in Example 2 was 128%, and the amount of mitochondria in Example 3 was 172%, relative to Control (see Fig. 2(b)).

The amount of mitochondria in Example 1 cultured for 21 days was 246% relative to Control (see Fig. 2(c)).

The amount of mitochondria in Example 1 cultured for 21 days was 115%, the mount of mitochondria in Example 2 was 124%, and the amount of mitochondria in Example 3 was 178%, relative to Control (see Fig. 2(c)).

According to these results, it turned out that resveratrol has an effect of increasing an amount of mitochondria.

It also turned out that the grape seed-derived anti-aging ingredients of this invention has an effect of increasing an amount of mitochondria.

Fig.3 shows a graph indicating a workload of a certain amount of mitochondria measured in Examples 1-3, Comparative Example 1, and Control.

In Fig.3, (a) shows a workload of a certain amount of mitochondria in cells cultured for 7 days, (b) shows a workload of a certain amount of mitochondria in cells cultured for 14 days, and (c) shows a workload of a certain amount of mitochondria in cells cultured for 21 days.

In Fig.3, assuming that the workload of a certain amount of the mitochondria in a Control is 100 %, the workload of a certain amount of the mitochondria in Examples 1-3 and Comparative Example 1 is shown in a ratio to the workload of a certain amount of mitochondria in the Control.

The workload of a certain amount of mitochondria was 22% relative to Comparative Example 1 cultured for 7 days (see Fig. 3(a)).

The workload of a certain amount of mitochondria in Example 1 cultured for 7 days was 74%, the workload of a certain amount of mitochondria in Example 2 was 58%, and the workload of a certain amount of mitochondria was 38% in Example 3, relative to Control (see Fig. 3(a)).

The workload of a certain amount of mitochondria in Comparative Example 1 cultured for 14 days was 20% relative to Control (see Fig. 3(b)).

The workload of a certain amount of mitochondria in Example 1 cultured for 14 days was 77%, the workload of a certain amount of mitochondria in Example 2 was 63%, and the workload of a certain amount of mitochondria in Example 3 was 34%, relative to Control (see Fig. 3(b)).

The workload of a certain amount of mitochondria in Comparative Example 1 cultured for 21 days was 49% relative to Control (see Fig.3(c)).

The workload of a certain amount of mitochondria in Example 1 cultured for 21 days was 88%, the workload of a certain amount of mitochondria in Example 2 was 76%, and the workload of a certain amount of mitochondria in Example 3 was 62%, relative to Control (see Fig.3(c)).

These results showed that resveratrol had an effect of decreasing the workload of a certain amount of mitochondria.

Moreover, it turned out that the grape seed-derived anti-aging ingredient according to the present invention also had the effect of decreasing the workload of a certain amount of mitochondria.

The result of Example 4 showed that the grape seed-derived anti-aging ingredient according to the present invention had the effect of increasing the amount of mitochondria and decreasing the workload of a certain amount of mitochondria.

With the increase in the amount of mitochondria, an amount of ATP (namely, workload) to be produced in a certain amount of mitochondria decreases. It is considered that the decrease in the workload of a certain amount of mitochondria, which was confirmed in Example 4, is due to the increased amount of mitochondria and the decreased amount of the production of ATP per certain amount of mitochondria.

This spares processing capability of mitochondria, decreases a production of active oxygen generated by mitochondria, and increases a production of superoxide dismutase (SOD) which is an enzyme to remove active oxygen, thereby reducing the amount of the active oxygen which leads to the damage of mitochondria and cells. Since about 90% of active oxygen is generated by mitochondria, it can be considered that decreasing the amount of the active oxygen produced by mitochondria with the grape seed-derived anti-aging ingredient according to the present invention can protect cells and organs, etc. consisting of cells from a damage and bring anti-aging effect.

### <Example 5: Test for measuring apoptosis and necrosis>

Next, an effect on the amount of apoptosis cells and the amount of necrosis cells in Examples and Comparative Example were confirmed.

Conventionally, since it has been thought that resveratrol has anti-aging effect by inducing apoptosis, the effect of the grape seed-derived anti-aging ingredient according to the present invention on the amount of the apoptosis cells was confirmed.

In addition, an effect of the grape seed-derived anti-aging ingredient according to the present invention on the amount of the necrosis cells which is the same cell death as the apoptosis was also confirmed.

The amount of the apoptosis cells was measured using Annexin V which has been conventionally used as an apoptosis marker. In more detail, the amount of the apoptosis cells was measured by selectively fluorescently-staining the apoptosis cells by fluorescently labeled Annexin V, using its property that Annexin V binds to phosphatidylserine often appearing on a surface of the apoptosis cells.

The amount of the necrosis cells was measured using an ethidium homodimer III (EthD-III) which has been conventionally used as a necrosis marker. In more detail, EthD-III is a film-impermeable nucleic acid probe and the amount of the necrosis cells was measured by using its property that neither the viable cells nor the apoptosis cells are stained but the necrosis cells are stained in strong red with EthD-III.

### (Experimental procedure)

After removing the culture supernatant of a normal human fibroblast cultured in Example 2, the normal human fibroblast was washed twice with PBS (NISSUI PHARMACEUTICAL CO., LTD., product No.:05913).

To the washed normal human fibroblast were added a 1×Binding Buffer containing a 1000-fold diluted Hoechist 33342 solution (DOJINDO LABORATORIES, product No.: 346-079511), a 20-fold diluted FITC-Annexin V, and a 20-fold diluted EthD-III. Then, the normal human fibroblast was cultured at 37 °C for 30 minutes.

Then, after removing culture supernatant, the normal human fibroblast was washed twice in the 1×Binding Buffer.

New 1×Binding Buffer was added to the normal human fibroblast after the washing, and images of nuclear staining (viable cell), Annexin V staining (apoptosis), and an EthD-III staining (necrosis) were taken by a fluorescence microscope.

The amount of apoptosis cells and the amount of necrosis cells were calculated from the fluorescent observation images.

Furthermore, FITC-Annexin V, EthD-III and 1×Binding Buffer used in Examples were from Apoptotic/Necrotic/Healthy Cells Detection Kit (Takara Bio, Inc. product No.: D25517).

### (Result)

Fig. 4 is a graph showing the amount of apoptosis cells measured in Examples 1-3, Comparative Example 1, and a Control.

In Fig. 4, (a) shows the amount of the apoptosis cells in cells cultured for 7 days, (b) shows the amount of the apoptosis cells in cells cultured for 14 days, and (c) shows the amount of the apoptosis cells in cells cultured for 21 days. Here, in Fig. 4, assuming that the amount of the apoptosis cells in the Control is 100%, and the amount of apoptosis cells in Examples 1-3 and Comparative Example is shown in a ratio to the amount of the apoptosis cell in the Control.

Compared to the Control, the amount of the apoptosis cells cultured for 7 days in Comparative Example 1 was 2184% (see Fig. 4 (a)).

On the other hand, compared to the Control, the amount of the apoptosis cells cultured for 7 days in Example 1 was 164%, the amount of the apoptosis cells in Example 2 was 102%, and the amount of the apoptosis cells in Example 3 was 483% (see Fig. 4 (a)).

Compared to the Control, the amount of the apoptosis cells cultured for 14 days in Comparative Example 1 was 1400% (see Fig. 4 (b)).

On the other hand, compared to the Control, the amount of the apoptosis cells cultured for 14 days in Example 1 was 107%, the amount of the apoptosis cells in the Example 2 was 313%, and the amount of the apoptosis cells in Example 3 was 226% (see Fig. 4 (b)).

Compared to the Control, the amount of the apoptosis cells in Comparative Example 1 cultured for 21 days was 4525% (see Fig. 4 (c)).

On the other hand, compared to the Control, the amount of the apoptosis cells cultured for 21 days in Example 1 was 59%, the amount of the apoptosis cells in Example 2 was 558%, and the amount of the apoptosis cells in Example 3 was 668% (see Fig. 4 (c)).

These results showed that resveratrol had an effect of increasing the amount of the apoptosis cells.

Moreover, it turned out that the grape seed-derived anti-aging ingredient according to the present invention also has the effect of increasing the amount of the apoptosis cells.

Fig. 5 is a graph showing the amount of necrosis cells measured in Examples 1-3, Comparative Example 1, and Control.

In Fig. 5, (a) shows the amount of the necrosis cells in cells cultured for 7 days, (b) shows the amount of the necrosis cells in cells cultured for 14 days, and (c) shows the amount of the necrosis cells in cells cultured for 21 days. In addition, in Fig. 5, the amount of the necrosis cell in the Control is 100%, and the amount of the necrosis cell of the Examples 1-3 and the Comparative Example is shown as a percentage to the amount of the necrosis cell in the Control.

Compared to the Control, the amount of the necrosis cells cultured for 7 days in Comparative Example 1 was 125% (see Fig. 5 (a)).

On the other hand, compared to the Controls, the amount of the necrosis cells cultured for 14 days in Example 1 was 47%, the amount of the necrosis cells in Example 2 was 47%, and the amount of the necrosis cells in Example 3 was 100% (see Fig. 5 (a)).

Compared to the Control, the amount of the necrosis cells cultured for 14 days in Comparative Example 1 was 801% (see Fig. 5 (b)).

On the other hand, compared to the Control, the amount of the necrosis cells cultured for 14 days in Example 1 was 109%, the amount of the necrosis cells in Example 2 was 108%, and the amount of the necrosis cells in Example 3 was 95% (see Fig. 5 (b)).

Compared to the Control, the amount of the necrosis cells cultured for 21 days in Comparative Example 1 was 608% (see Fig. 5 (c)).

On the other hand, compared to the Control, the amount of the necrosis cells cultured for 21 days in Example 1 was 55%, the amount of the necrosis cells in Examination 2 was 28%, and the amount of the necrosis cells in Example 3 was 24% (see Fig. 5 (c)).

These results showed that resveratrol has an effect of increasing the amount of the necrosis cells in all the periods.

On the other hand, it turned out that the grape seed-derived anti-aging ingredient according to the present invention has the effect of decreasing the amount of the necrosis cells.

The result of the Example 5 showed that the grape seed-derived anti-aging ingredient according to the present invention has an effect of increasing the amount of the apoptosis cells and decreasing the amount of the necrosis cells.

Apoptosis is a mechanism which induces cell death to a harmful cell or a cell unnecessary to an organism and removes such cells, thereby leading to generation of new cells by promptly removing aged cells and disabled cells. The apoptosis normally functions to remove senescent cells, resulting in increase in the healthy cells in a cell level. An organ consisting of healthy cells is young.

Moreover, an apoptosis also leads to removal of immune cells causing an autoimmune reaction which is a mechanism of autoimmune disease, and it can be expected to reduce autoimmune disease by its correct functioning.

On the other hand, necrosis has an adverse influence, such as inflammatory reaction on surrounding tissues, since cells explode without pre-processing an intracellular enzyme, etc., resulting in extracellularly releasing of a cell content containing enzyme, etc. This adverse influence promotes deterioration of cells and causes inflammation in a body which increases as aging. Thus, necrosis causes a damage to cells by adversely affecting on surrounding tissues, such as inflammatory reaction.

Therefore, necrosis promotes aging of cells.

Consequently, it is extremely effective to promote apoptosis and suppress necrosis, in order to suppress aging of cells.

For that reason, it is understood that the grape seed-derived anti-aging ingredient according to the present invention having an effect of promoting apoptosis and suppressing necrosis has remarkable anti-aging effect.

On the other hand, it turned out that resveratrol promotes not only apoptosis but also necrosis.

As described above, the suppression of necrosis is required to suppress aging of cells. Therefore, it is considered that resveratrol promoting not only apoptosis but also necrosis has less anti-aging effect than the grape seed-derived anti-aging ingredient according to the present invention.

### <Example 6: Toxicity test >

In order to confirm toxicity of the grape seed-derived anti-aging ingredient according to the present invention, an acute oral toxicity test (limit test) (based on OECD Guidelines for the Testing of Chemicals 420 (2001)) using female mice was performed.

### (Test method)

Powdered grape seed-derived anti-aging ingredient according to the present invention was suspended with water for injection to prepare a test solution of 100mg/mL.

ICR strain five-week-old female mice were purchased from Japanese SLC, Inc. and preliminary bred for about one week. After confirming that there was no abnormality in general status, they were used for the test.

Five female mice to be used for the test were housed in a polycarbonate cage, respectively and were fed in a breeding room set at a room temperature (23 °C ±2 °C), and at a lighting time of 12 hours/day.

The mice were fed a laboratory chow (Nosan Corporation, a product name: lab MR stock (a pellet for mouse, rat)) and drinking water (tap water).

A test group to be administered 2000mg/kg of grape seed-derived anti-aging ingredient and the control group to be administered water for injection as a solvent control were set up, and five female mice were used for each group respectively.

The female mice used for the test were fasted for about 4 hours before the administration.

After measuring weight of each female mouse, the test group and the control group were forced to be orally administered test solution and water for injection at a single dose of 20 mL/kg, respectively, using a gastric tube.

The female mice were observed for 14 days after the administration.

The female mice were observed frequently on the day of the administration and once a day from the following day.

The weight of the female mice was measured on Day 7 and Day 14 after the administration, and the groups were compared by t-test at 5% of significance level.

All female mice were autopsied after the end of an observation period.

### (Result)

In any administration groups, no deaths were reported during the observation period.

In any administration groups, no abnormality was found during the observation period.

Table 1 shows the result of weight change of the test group and the control group.

In the measurement of body weight on Day 7 and Day 14 after the administration, it turned out that the test group did not have a significant difference in weight compared to the control group.

No abnormality was found in all female mice in the autopsy after the end of the observation period.

**[Table 1]**

| Treatment Groups | Weight before the administration | Weight after the administration | |
|---|---|---|---|
| | | Day 7 after the administration | Day 14 after the administration |
| Test Group | 27. 4±1. 1 (5) | 29. 0±1.0 (5) | 30. 8±1. 7(5) |
| Control Group | 27. 0±1.1 (5) | 29. 4±2.1 (5) | 30. 2±2. 5 (5) |

Weight is shown by average value ± standard deviation (unit: g).

The number of the female mice whose weight was measured is shown in parentheses.

These results showed that in the single-dose oral administration to female mice, LD50 value of the grape seed-derived anti-aging ingredient according to the present application exceeded 2000mg /kg in the female mice.

The results of Examples 3-5 showed that the grape seed-derived anti-aging ingredient according to the present invention increases the amount of mitochondria, reduces the workload of a certain amount of mitochondria, increases the amount of apoptosis cells, and decreases the amount of necrosis cells, and thus has remarkable anti-aging effect.

Moreover, as a result of comparison to the resveratrol alone expected to have anti-aging effect, it turned out that the grape seed-derived anti-aging ingredient according to the present invention had much more remarkable anti-aging effect than the resveratrol alone.

In addition, the result of Example 6 showed that the grape seed-derived anti-aging ingredient according to the present invention had lower cytotoxicity and more remarkable safety.

Thus, the health food and cosmetics containing the grape seed-derived anti-aging ingredient according to the present invention have remarkable anti-aging effect and safety.

### [INDUSTRIAL APPLICABILITY]

According to the present invention, since the anti-aging health food and cosmetics contain the grape seed-derived anti-aging ingredient consisting of 60 wt. % or more by weight of crudely-purified grape seed-derived polyphenol, the anti-aging health food and cosmetics having remarkable anti-aging effect can be provided.

Therefore, the present invention is suitably used as health food and cosmetics having anti-aging effect.

## Claims

1. (Currently Amended) Health food and cosmetics for increasing the amount of apoptosis cells and decreasing the amount of necrosis cells, comprising a sprout-forcing grape seed-derived ingredient consisting of 60 wt. % or more of crudely-purified and sprout-forcing grape seed-derived polyphenol.

2. (Currently Amended) A method of manufacturing a sprout-forcing grape seed-derived ingredient for increasing the amount of apoptosis cells and decreasing the amount of necrosis cells, comprising:
(Step 1) immersing one or more grape seeds selected from a group consisting of Vitis vinifera L., Vitis labrusca L., Vitis coignetiae L., Vitis amurensis L., and Vitis shiragai L.
into water at 30 °C - 60 °C for 20-80 hours;
(Step 2) removing from the water the grape seed immersed in said Step 1 and naturally drying it in the air;
(Step 3) immersing the grape seed naturally dried in said Step 2 in water at 15 °C - 45 °C for 10-100 minutes;
(Step 4) removing from the water the grape seed immersed in said Step 3 and naturally drying it in the air;
(Step 5) repeating said Steps 3 and 4 to force sprouting of the grape seed until an embryonic portion of the grape seeds naturally dried in said Step 4 will slightly bulge and swell;
(Step 6) drying the sprout-forcing grape seed at 35 °C -60 °C;
(Step 7) powdering the grape seed dried in said Step 6;
(Step 8) obtaining an extracted fraction containing a grape seed-derived polyphenol by immersing the powdered grape seed obtained in said Step 7 in either of water, ethanol, or a mixed solvent of water and ethanol; and
(Step 9) drying and powdering the extracted fraction containing the grape seed-derived polyphenol obtained in said Step 8.

3. The health food and cosmetics according to claim 1 and the method according to claim 2, wherein said grape seed is a seed of one or more wine grape cultivars selected from a group consisting of: Agiorgitiko, Viognier, CabernetSauvignon, Cabernet Franc, Gamay, Carignan, Carmenere, Xinomavro, Grenache, Gewurztraminer, Kerner, Colombard, Koshu, Sultana, Sangiovese, Chardonnay, Chenin Blanc, Syrah, Zinfandel, Semillon, Sauvignon Blanc, Tannat, Zweigelt, Tempranillo, Trebbiano, Nebbiolo, Nero D'Avola, Barbera, Pinotage, PinotNoir, Pinot Gris, Pinot Blanc, Petit Verdot, Black Queen, Muscat Bailey A, Malbec, Muller-Thurgau, Mourvedre, Meunier, Melon de Bourgogne, Merlot, Moscato, Yama-Sauvignon, Riesling, and Ruby Cabernet.

## Patentansprüche

1. Reformkost und Kosmetika zum Erhöhen der Menge an Apoptosezellen und Verringern der Menge an Nekrosezellen, umfassend einen aus Traubenkernen gewonnenen, keimtreibenden Inhaltsstoff, bestehend aus 60 Gew.-% oder mehr von roh gereinigtem und keimtreibendem aus Traubenkernen gewonnenem Polyphenol.

2. Verfahren zum Herstellen eines keimtreibenden, aus Traubenkernen gewonnenen Inhaltsstoff zum Erhöhen der Menge an Apoptosezellen und Verringern der Menge an Nekrosezellen, umfassend:
(Schritt 1) Eintauchen eines oder mehrerer Traubenkerne, ausgewählt aus einer Gruppe bestehend aus Vitis vinifera L., Vitis labrusca L., Vitis coignetiae L., Vitis amurensis L. und Vitis shiragai L. in Wasser bei 30 °C - 60 °C für 20-80 Stunden;
(Schritt 2) Entfernen des in Schritt 1 eingetauchten Traubenkerns aus dem Wasser und natürliches Trocknen an der Luft;
(Schritt 3) Eintauchen des in Schritt 2 natürlich getrockneten Traubenkerns in Wasser bei 15 °C - 45 °C für 10-100 Minuten;
(Schritt 4) Entfernen des in Schritt 3 eingetauchten Traubenkerns aus dem Wasser und natürliches Trocknen an der Luft;
(Schritt 5) Wiederholen der Schritte 3 und 4, um das Keimen des Traubenkerns zu erzwingen, bis ein embryonaler Abschnitt der in Schritt 4 natürlich getrockneten Traubenkerne leicht ausbaucht und anschwillt;
(Schritt 6) Trocknen der keimtreibenden Traubenkerne bei 35 °C - 60 °C;
(Schritt 7) Pulverisieren des in Schritt 6 getrockneten Traubenkerns;
(Schritt 8) Erhalten einer extrahierten Fraktion, die ein von Traubenkernen abgeleitetes Polyphenol enthält, durch Eintauchen des in Schritt 7 erhaltenen pulverisierten Traubenkerns in entweder Wasser, Ethanol oder ein gemischtes Lösungsmittel aus Wasser und Ethanol; und
(Schritt 9) Trocknen und Pulverisieren der extrahierten Fraktion, die das in Schritt 8 erhaltene aus Traubenkernen gewonnene Polyphenol enthält.

3. Reformkost und Kosmetika nach Anspruch 1 und Verfahren nach Anspruch 2, wobei der Traubenkern ein Kern von einer oder mehreren Keltertraubensorten ist, ausgewählt aus einer Gruppe bestehend aus: Agiorgitiko, Viognier, Cabemet Sauvignon, Cabernet Franc, Gamay, Carignan, Carmenere, Xinomavro, Grenache, Gewürztraminer, Kerner, Colombard, Koshu, Sultana, Sangiovese, Chardonnay, Chenin Blanc, Syrah, Zinfandel, Semilion, Sauvignon Blanc, Tannat, Zweigelt, Tempranillo, Trebbiano, Nebbiolo, Nero D'Avola, Barbera, Pinotage, Pinot Noir, Grauburgunder, Weißburgunder, Petit Verdot, Black Queen, Muscat Bailey A, Malbec, Müller-Thurgau, Mourvedre, Meunier, Melon de Bourgogne, Merlot, Moscato, Yama-Sauvignon, Riesling und Ruby Cabernet.

## Revendications

1. Aliments diététiques et cosmétiques pour augmenter la quantité de cellules d'apoptose et diminuer la quantité de cellules de nécrose, comprenant un ingrédient dérivé de pépins de raisin de germination forcée constitué de 60 % en poids ou plus de polyphénols dérivés de pépins de raisin grossièrement purifiés et de germination forcée.

2. Procédé de fabrication d'un ingrédient dérivé de pépins de raisin de germination forcée pour augmenter la quantité de cellules d'apoptose et diminuer la quantité de cellules de nécrose, comprenant :
(Étape 1) immerger un ou plusieurs pépins de raisin choisis dans un groupe constitué de *Vitis vinifera* L., *Vitis labrusca* L., *Vitis coignetiae* L., *Vitis amurensis* L. et *Vitis shiragai* L. dans de l'eau à 30 °C - 60 °C pendant 20-80 heures ;
(Étape 2) sortir de l'eau les pépins de raisin immergés dans ladite Étape 1 et les sécher naturellement à l'air ;
(Étape 3) immerger les pépins de raisin séchés naturellement dans ladite Étape 2 dans de l'eau à 15 °C -45 °C pendant 10-100 minutes ;
(Étape 4) sortir de l'eau les pépins de raisin immergés dans ladite Étape 3 et les sécher naturellement à l'air ;
(Étape 5) répéter lesdites Étapes 3 et 4 pour forcer la germination des pépins de raisin jusqu'à ce qu'une partie embryonnaire des pépins de raisin séchés naturellement dans ladite Étape 4 soit légèrement bombée et gonflée ;
(Étape 6) sécher les pépins de raisin en voie de germination à 35 °C - 60 °C;
(Étape 7) pulvériser les pépins de raisin séchés dans ladite Étape 6 ;
(Étape 8) obtenir une fraction extraite contenant un polyphénol dérivé de pépins de raisin en immergeant la poudre de pépins de raisin obtenue dans ladite Étape 7 soit dans de l'eau, de l'éthanol, ou un solvant mixte d'eau et d'éthanol ; et
(Étape 9) séchage et pulvérisation de la fraction extraite contenant le polyphénol dérivé de pépins de raisin obtenu dans ladite Étape 8.

3. Aliments diététiques et cosmétiques selon la revendication 1 et procédé selon la revendication 2, dans lequel ledit pépin de raisin est un pépin d'un ou plusieurs cultivars de raisin de cuve choisis dans un groupe consistant en : Agiorgitiko, Viognier, CabemetSauvignon, Cabernet Franc, Gamay, Carignan, Carmenere, Xinomavro, Grénache, Gewurztraminer, Kerner, Colombard, Koshu, Sultana, Sangiovese, Chardonnay, Chenin Blanc, Syrah, Zinfandel, Semilion, Sauvignon Blanc, Tannat, Zweigelt, Tempranillo, Trebbiano, Nebbiolo, Nero D'Avola, Barbera, Pinotage, Pinot Noir, Pinot Gris, Pinot Blanc, Petit Verdot, Black Queen, Muscat Bailey A, Malbec, Muller-Thurgau, Mourvèdre, Meunier, Melon de Bourgogne, Merlot, Moscato, Yama-Sauvignon, Riesling et Ruby Cabernet.
